# FASCICULE DE BREVET EUROPEEN

(11) **EP 0 477 109 B1**
(45) Date de publication et mention de la délivrance du brevet: **11.06.1997**
(21) Numéro de dépôt: 91420306.2
(22) Date de dépôt: 02.09.1991
(51) Int. Cl.: A61F 2/16

(54) **Implant intra-oculaire pour correction de la myopie**
Intraokulares Implantat zur Myopiekorrektur
Intraocular implant for correcting myopia

(30) Priorité: 04.09.1990 FR 9011236
(43) Date de publication de la demande: 25.03.1992
(73) Titulaire: LABORATOIRES DOMILENS SOCIETE ANONYME, 69007 Lyon (FR)
(72) Inventeur: Baikoff, Georges, F-13009 Marseille (FR); Subrin, Philippe, F-38300 Domarin (FR)
(74) Mandataire: Guerre, Dominique

(56) Documents cités:
- EP-A- 0 346 245
- WO-A-89/06115
- GB-A- 2 215 076
- US-A- 4 666 446
- US-A- 4 769 035

## Description

La présente invention concerne un implant intra-oculaire destiné à être inséré dans la chambre antérieure de l'oeil, par chirurgie ophtalmologique, pour corriger la myopie du patient sans ablation du cristallin.

Selon le document EP-A-0 346 245, on a décrit et proposé un implant tel que précédemment défini, composé :
- d'une partie optique comprenant pour l'essentiel une lentille divergente de correction à puissance optique négative, par exemple une lentille bi-concave, et dont par conséquent l'épaisseur croît en général de son axe optique vers sa périphérie, cette dernière présentant alors un bord d'épaisseur relativement importante
- et d'une partie haptique, comprenant par exemple deux anses de support en vis-a-vis, pour le support dans la chambre antérieure de l'oeil corrigé de la partie optique, de part et d'autre de cette dernière.

On rappelle que selon la terminologie consacrée dans le domaine technique de la présente invention, l'expression "optique" ou "partie optique" désigne de manière générale la partie de l'implant traversée par les rayons lumineux passant par la pupille, quelque soit le degré de dilatation de cette dernière. Tout ou partie de cette optique peut être occupé par la lentille de correction proprement dite, de puissance optique appropriée à la correction souhaitée.

L'expression "haptique" ou "partie haptique" désigne quant à elle la partie de l'implant n'ayant pour l'essentiel aucun rôle ou utilité vis-a-vis des rayons lumineux passant par la pupille, et assurant de manière appropriée le support, la mise en place, et le positionnement de la partie optique dans le logement anatomique de l'oeil retenu pour le recevoir.

Comme on le sait, la partie haptique et la partie optique peuvent être distinctes, ou obtenues de manière monobloc en un seul et même matériau, par exemple une matière plastique appropriée.

Selon le document EP-A-0 346 245, la partie optique se confond pratiquement en totalité avec la lentille de correction, laquelle peut alors présenter un diamètre relativement important, au moins égal à 4,5 mm, couvrant mieux le champ visuel de la pupille.

Ceci étant, l'expérience accumulée sur l'acceptation par le patient de tels implants a révélé la difficulté suivante.

Sous faible éclairement reçu par l'oeil du patient, ce dernier perçoit un halo lumineux diffus, perturbant la perception distincte d'une image, nonobstant la bonne correction obtenue avec la partie optique de l'implant.

Le Déposant s'est alors livré à l'analyse optique du défaut ainsi constaté, détaillée ci-après, pour identifier l'origine du halo précité ; et c'est cette origine dûment reconnue qui est à la base de la présente invention.

Sous faible éclairement, par exemple en vision nocturne, la pupille du patient se trouve alors dilatée selon une section sensiblement circulaire relativement importante. Cette section peut être supérieure à la surface concentrique ou coaxiale avec l'axe optique de l'oeil, de la partie optique de l'implant, de telle sorte qu'il existe alors dans la chambre antérieure de l'oeil un interstice annulaire non occulté par cette même partie optique. Par cet interstice, un rayonnement ou lumière périphérique peut parvenir directement à l'iris de l'oeil du patient, c'est-à-dire sans absorption ou passage dans la partie optique de l'implant.

Une faible partie de ce rayonnement périphérique se réfléchit sur la bordure relativement épaisse de la partie optique, agissant comme un miroir, et arrive de manière diffuse sur la rétine.

Une partie plus importante du rayonnement périphérique passe directement dans l'iris, et arrive défocalisée sur la rétine, c'est-à-dire focalisée en avant de la rétine, compte tenu de la myopie du patient.

Au total, du fait de ce rayonnement périphérique, la rétine reçoit une pluralité de taches se superposant les unes aux autres, correspondant à des images défocalisées de manière différente, et générant ensemble un halo lumineux, certes de faible intensité, mais "parasitant" en quelque sorte l'image focalisée sur la rétine, obtenue avec la lentille de correction.

Il n'apparaît pas possible d'augmenter le diamètre de la partie optique, et partant de la lentille de correction, car ceci conduit immédiatement à un accroissement de l'épaisseur du bord de cette même partie optique. Un tel accroissement risquerait de provoquer des contacts implant/endothelium cornéen, la profondeur de la chambre antérieure de l'oeil phake étant relativement réduite.

A partir de cette analyse, la présente invention a pour objet de maîtriser ou contrôler ce rayonnement périphérique, à la lisière de la lentille de correction, pour le transformer sur la rétine en un signal lumineux compatible avec l'image due à la lentille de correction.

Selon la présente invention, ce résultat est obtenu par la coopération des trois choix conceptionnels suivants :
1) l'extension radiale de la lentille de correction, à partir de son centre ou de son axe optique, est limitée par un cercle de diamètre compris entre 3,5 et 4,5 mm ; on fait donc le choix d'une lentille de correction, inscrite dans un cercle de diamètre relativement plus faible que celui ou ceux envisagés dans le document EP-A-0 346 245
2) l'extension radiale de la partie optique, a partir de son centre, ou de l'axe optique de la lentille de correction, est augmentée jusqu'à un cercle de diamètre compris entre 4,8 et 6,5 mm ; on fait donc le choix d'une partie optique, inscrite dans un cercle de diamètre relativement plus large que celui ou ceux envisagés dans le document EP-A-0 346 245, moyennant quoi on dégage ou ménage, autour de la lentille de correction, une bordure traversée par la lumière périphérique pouvant passer par la pupille
3) cette bordure périphérique est conformée selon une lentille convergente, de puissance positive comprise entre 20 et 60 dioptries, pour focaliser la lumière périphérique en avant de la rétine.

Grâce à l'invention, la lumière ou le rayonnement périphérique discuté précédemment se trouve contrôlé, a savoir focalisé, sur une zone d'extension axiale limitée, très en avant de la rétine, moyennant quoi cette dernière reçoit une tache lumineuse relativement homogène et régulière, de faible intensité lumineuse puisque très défocalisée, entourant l'image focalisée par la lentille de correction, dans une zone rétinienne de faible acuité visuelle. L'expérience montre que dans ces conditions, il est alors beaucoup plus facile a l'oeil du patient de discriminer ou distinguer l'image corrigée.

Les choix dimensionnels selon l'invention permettent de situer la bordure convergente, dans un anneau de diamètre intérieur égal à 3,5 mm et de diamètre extérieur égal a 6,5 mm. Le choix des dimensions effectives de la bordure convergente, comme de celles de l'implant, dépend pour partie de la myopie à corriger et de la configuration de l'oeil du patient. Ceci étant, pour cette bordure convergente, l'expérience a montré qu'il était difficile de descendre au-dessous de 3,5 mm, sans affecter la correction apportée par la lentille, et d'aller au-dessus de 6,5 mm, sans risquer un contact implant/endothelium cornéen.

S'agissant de la puissance optique de la bordure de focalisation, l'expérience a aussi montré qu'en dessous de 20 dioptries, on rapproche trop de la rétine le point de focalisation du rayonnement périphérique, de telle sorte que la tache lumineuse devient perceptible par la rétine. Et au-dessus de 60 dioptries, le rayon de courbure de la bordure de focalisation devient difficilement raccordable avec celui de la lentille, et comme dit précédemment, l'épaississement du bord de la lentille majore le risque de contact avec l'endothelium cornéen.

Conformément au document US-A-4 666 446, et plus précisément le mode d'exécution décrit par référence aux figures 13 à 15, et conformément au document WO-A-89 06115, et plus précisément au mode d'exécution représenté aux figures 5 et 6, on a proposé et décrit avec une fonction totalement différente, et dans une autre application, un implant intra-oculaire comprenant :
- une partie optique constituée par une lentille centrale divergente, à puissance optique négative, dont l'épaisseur croît radialement à partir de son centre optique, et par une bordure périphérique conformée selon une lentille convergente
- et une partie haptique pour le support de la partie optique, de part et d'autre de cette dernière.

Un tel implant intra-oculaire prescrit et utilisé pour remédier à la dégénérescence maculaire, est disposé dans la chambre postérieure de l'oeil, et par conséquent derrière la pupille, après ablation du cristallin, et donc en remplacement de ce dernier. Cet implant a pour fonction de permettre au patient deux types de vision, à savoir :
- uniquement grâce à la lentille périphérique convergente de la partie optique, une vision non grossie, avec un champ angulaire non limité
- grâce à la lentille centrale divergente de la partie optique, et en association avec une lunette convergente portée par le patient, et à la manière d'un télescope de Gallilée dont la partie divergente est dans l'oeil et la partie convergente à l'extérieur de l'oeil, une vision grossie, mais avec un champ angulaire limité.

Les implants décrits dans les documents US-C-4 666 446 et WO-A-89 06115 sont donc totalement différents de ceux selon la présente invention, dans leur fonction, leur usage et application ophtalmologique.

Plus précisément, les implants selon l'invention se distinguent de ceux décrits par cet art antérieur, en ce qu'ils ont pour fonction de corriger la myopie, sont agencés pour être disposés dans la chambre antérieure de l'oeil, sans ablation du cristallin, et en ce que dans ces conditions la lentille centrale divergente est une lentille de correction, et la lentille convergente est une lentille de focalisation de la lumière périphérique en avant de la rétine.

Selon la présente invention, la partie optique peut avoir toute forme appropriée, circulaire par exemple, la définition précédente précisant seulement la limite externe dans laquelle doit s'inscrire cette partie optique. Il en est de même pour la lentille de correction, la définition précédente précisant seulement la limite externe dans laquelle doit s'inscrire cette lentille.

Par ailleurs, de manière déjà connue en soi, la lentille de correction peut être composée d'au moins deux lentilles partielles, toujours divergentes, mais de puissances optiques ou focales respectivement différentes, ces lentilles partielles étant disposées dans la partie optique de toute manière appropriée, notamment de manière diamétralement opposée, concentrique, etc... En cas de pluralité de lentilles partielles, la variation de leurs puissances optiques négatives et respectives peut être obtenue de manière quasiment continue, de telle sorte que la lentille de correction présente une face antérieure et/ou une face postérieure de profil régulier. La lentille de correction et la bordure de focalisation peuvent être obtenues dans des matériaux respectivement différents, selon l'indice de réfraction désiré.

La présente invention apporte en outre les avantages déterminants suivants.

Pour une même puissance optique de la lentille de correction, la partie optique de l'implant se trouve écartée de l'endothelium de l'oeil d'une distance au moins égale à 0,5 mm, par rapport à un implant selon le document EP-A-0 346 245. Ceci résulte de tout ou partie des choix conceptionnels précédents :
- en limitant l'extension radiale de la lentille de correction, on limite l'épaisseur de sa bordure, tout en limitant sa hauteur dans la chambre antérieure
- la bordure périphérique de focalisation présente en section un profil convergent, ce qui limite l'extension radiale de la partie optique.

Tout ceci favorise, et la bonne mise en place de l'implant dans la chambre antérieure de l'oeil, c'est-à-dire sans endommager l'endothelium, et une position de l'implant préservée de tout contact avec l'endothelium pendant l'activité du patient implanté.

Par ailleurs, la forme convergente de la bordure de focalisation confère à la partie optique une forme beaucoup plus anatomique par rapport à la chambre antérieure de l'oeil. Ceci permet de compenser en quelque sorte la forme de la lentille de correction, opposée à celle de cette même chambre.

La présente invention est maintenant décrite par référence aux dessins annexés, dans lesquels :
- la figure 1 représente une vue de face d'un implant intra-oculaire selon la présente invention
- la figure 2 représente une vue de côté du même implant
- la figure 3 représente le schéma optique de correction obtenue avec un implant selon la présente invention.

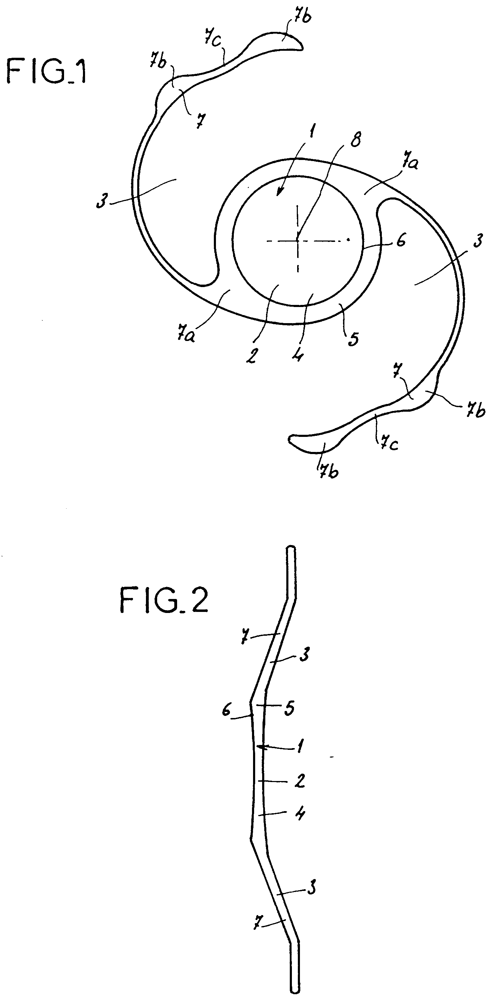

Conformément aux figures 1 et 2, l'implant 1 selon l'invention comprend de manière générique, une partie optique 2, et une partie haptique3. La partie optique 2 comprend, d'une part une lentille 4 divergente de correction de la myopie, bi-concave, et par conséquent à puissance optique négative (comprise entre -5 et -30 dioptries), et de profil circulaire, et d'autre part une bordure 5 annulaire, conformée selon un ménisque ou lentille convergente, et par conséquent présentant une face antérieure convexe, ménageant une zone d'inflexion 6 avec la face antérieure concave de la lentille 4, et une face postérieure concave, située approximativement dans le prolongement de la face postérieure concave de la même lentille 4. Quant à la partie haptique 3, elle est constituée de deux anses 7 en vis-à-vis, comportant chacune à une extrémité une zone d'attache 7a, sur la bordure de focalisation 5, et à l'autre extrémité libre une zone intégrée d'appui, constituée par deux patins 7b de contact espacés l'un de l'autre. Comme le montre la figure 1, les deux anses 7 sont recourbées régulièrement vers la partie optique 2, de leurs zones d'attache 7a à leurs zones d'appui 7c, dans la même direction centripète, par rapport au centre ou axe optique 8 de l'implant.

Comme déjà dit, la bordure de focalisation 5, la lentille de correction 4 et la partie haptique 3 peuvent être obtenues de manière monobloc en un seul et même matériau, par exemple en PMMA (polyméthyl-métacrylate). On peut également retenir d'autres matériaux, présentant un indice de réfraction relativement élevé, compris par exemple entre 1,65 et 1,75, notamment en polysulfone, permettant pour une même puissance optique de correction de limiter encore l'épaisseur de la lentille 4, et partant sa hauteur ou extension radiale.

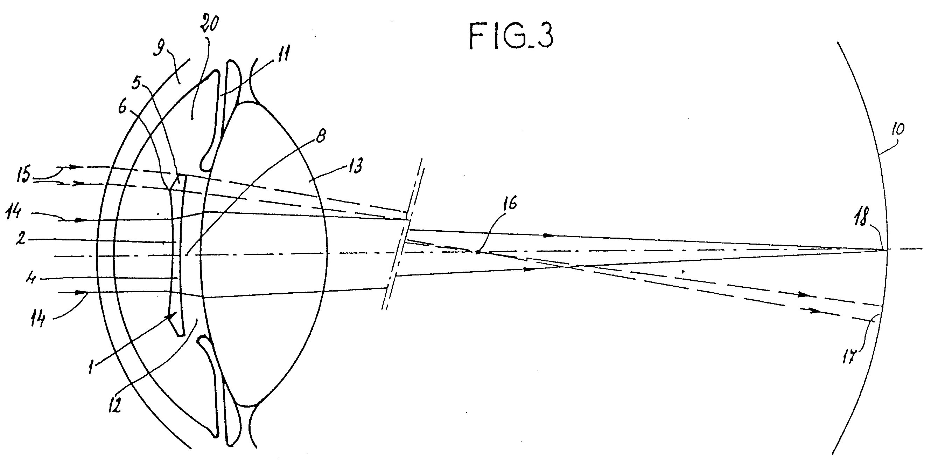

Les dimensions et autres paramètres géométriques de l'implant sont adaptés en général, d'une part à la myopie à corriger, et d'autre part a l'anatomie de l'oeil à implanter. Ceci étant, comme déjà dit, l'extension radiale de la lentille centrale 4 de correction est limitée par un cercle de diamètre compris entre 3,5 et 4,5 mm, tandis que l'extension radiale de la partie optique 2 ou bordure périphérique 5 est augmentée jusqu'à un cercle de diamètre compris entre 4,8 et 6,5 mm. Comme le montre la figure 2, une partie de l'haptique 3, et plus précisément une branche des anses 3 présente une angulation par rapport au plan de la partie optique 2, comprise entre 10 et 25° ; comme montré par référence a la figure 3, ceci permet de maintenir ou positionner l'implant à distance de la cornée, de la pupille, et du cristallin.

Conformément à la figure 3, on a représenté schématiquement l'oeil implanté, comprenant la cornée 9, la rétine 10, l'iris 11, la pupille 12 et le cristallin 13. L'opération ophtalmologique aboutit à la mise en place de l'implant 1 dans la chambre antérieure 20 selon l'axe optique de l'oeil, qui se confond avec celui de la partie optique 2, ou lentille 4. Compte tenu de la correction apportée par la lentille 4, un rayonnement 14 passant par le centre de la pupille 12 est focalisé en 18 sur la rétine 10. Sous faible éclairage, par exemple en vision nocturne, l'iris 11 est amené à se rétracter, comme montré à la figure 3 ; dans cette conformation, la partie optique 2 de l'implant occulte toujours la pupille 12, grâce aux choix conceptionnels selon l'invention. Toujours dans cette conformation, un rayonnement périphérique 15 est défocalisé par rapport à la rétine 10 et plus précisément focalisé au point 16, en avant de la rétine 10, de telle manière qu'une tache 17 de faible intensité lumineuse est obtenue sur la rétine 10. L'expérience démontre que l'image corrigée 18 peut être beaucoup mieux discriminée ou distinguée par l'oeil, par rapport à la tache 17, de telle sorte que le patient tolère beaucoup mieux un implant selon la présente invention.

## Revendications

1. Implant (1) intra-oculaire destiné à la correction de la myopie et agencé pour être disposé dans la chambre antérieure de l'oeil, sans ablation du cristallin, ledit implant étant constitué d'une partie optique (2) comprenant une lentille centrale divergente (4) à puissance optique négative dont l'épaisseur croit radialement à partir de son centre optique (8), et d'une partie haptique (3) pour le support de ladite partie optique, de part et d'autre de cette dernière, caractérisé en ce que la partie optique (2) comprend, outre la lentille centrale divergente (4), une bordure périphérique (5) conformée selon une lentille convergente, la lentille centrale divergente (4) étant une lentille de correction et la lentille périphérique convergente (5) étant une lentille de focalisation de la lumière périphérique en avant de la rétine.

2. Implant (1) selon la revendication 1, caractérisé en ce qu'en combinaison l'extension radiale de la lentille divergente centrale (4) est limitée par un cercle de diamètre compris entre 3,5 et 4,5 mm, l'extension radiale de la lentille convergente périphérique (5) est augmentée jusqu'à un cercle de diamètre compris entre 4,8 et 6,5 mm, pour occulter la rétine sous faible éclairage, et ladite lentille convergente périphérique (5) présente une puissance positive comprise entre 20 et 60 dioptries.

3. Implant (1) selon la revendication 1, caractérisé en ce qu'au moins la lentille de correction (4) est réalisée en un matériau présentant un indice de réfraction relativement élevé, compris entre 1,65 et 1,75, notamment en polysulfone.

4. Utilisation d'un implant (1) intra-oculaire comprenant une partie optique (2) constituée d'une lentille centrale divergente (4) à puissance optique négative, dont l'épaisseur croît radialement à partir de son centre optique (8), et d'une bordure périphérique (5) conformée selon une lentille convergente, et une partie haptique (3) pour le support de ladite partie optique (2), de part et d'autre de cette dernière, pour la réalisation d'un implant pour correction de la myopie, sans ablation du cristallin, conforme à la revendication 1.

## Claims

1. Intraocular implant (1), intended for the correction of myopia and designed to be arranged in the anterior chamber of the eye, without ablation of the crystalline lens, the said implant consisting of an optic part (2) comprising a central diverging lens (4) of minus optical power, whose thickness increases radially from its optical centre (8), and of a haptic part (3) for supporting said optic part, on each side of the latter, characterized in that the optic part (2) comprises, in addition to the central diverging lens (4), a peripheral edge (5) shaped in the manner of a converging lens, the central diverging lens (4) being a corrective lens, and the peripheral converging lens (5) being a lens for focusing the peripheral light in front of the retina.

2. Implant (1) according to Claim 1, characterized in that, in combination, the radial extension of the central diverging lens (4) is limited by a circle with a diameter of between 3.5 and 4.5 mm, the radial extension of the peripheral converging lens (5) is increased to a circle with a diameter of between 4.8 and 6.5 mm, in order to cover the retina in dim light, and said peripheral converging lens (5) has a plus power in the range between 20 and 60 diopters.

3. Implant (1) according to Claim 1, characterized in that at least the corrective lens (4) is made of a material having a relatively high refractive index, of between 1.65 and 1.75, in particular polysulfone.

4. Use of an intraocular implant (1), comprising an optic part (2) consisting of a central diverging lens (4) of minus optical power, whose thickness increases radially from its optical centre (8), and a peripheral edge (5) shaped in the manner of a diverging lens, and a haptic part (3) for supporting said optic part, for producing an implant for correction of myopia, without ablation of the crystalline lens, in accordance with Claim 1.

## Patentansprüche

1. Intraokulares Implantat (1) zur Korrektur der Kurzsichtigkeit, das dazu beschaffen ist, in der vorderen Augenkammer ohne Entfernung der Augenlinse angeordnet zu werden, wobei das Implantat aus einem optischen Abschnitt (2) mit einer mittigen Zerstreuungslinse (4) mit negativer Brechkraft, deren Dicke von ihrem optischen Mittelpunkt (8) aus radial zunimmt, und aus einem haptischen Abschnitt (3) zum Halten des optischen Abschnittes gebildet wird, der beidseits desselben angeordnet ist, dadurch gekennzeichnet, daß der optische Abschnitt (2) außer der mittigen Zerstreuungslinse (4) eine umfängliche Einfassung (5) aufweist, die einer Sammellinse entsprechend ausgebildet ist, wobei die mittige Zerstreuungslinse (4) eine Korrekturlinse und die umfängliche Sammellinse (5) eine Linse zum Fokussieren des Randzonenlichtes vor der Netzhaut ist.

2. Implantat (1) nach Anspruch 1, dadurch gekennzeichnet, daß in Kombination die radiale Ausdehnung der mittigen Zerstreuungslinse (4) durch einen Kreis mit einem Durchmesser zwischen 3,5 und 4,5 mm begrenzt ist, und die radiale Ausdehnung der umfänglichen Sammellinse (5) bis zu einem Kreis mit einem Durchmesser zwischen 4,8 und 6,5 mm weiterreicht, um die Netzhaut unter schwacher Beleuchtung abzudecken, und daß die umfängliche Sammellinse (5) eine positive Brechkraft zwischen 20 und 60 Dioptrien aufweist.

3. Implantat (1) nach Anspruch 1, dadurch gekennzeichnet, daß zumindest die Korrekturlinse (4) aus einem Material mit einem relativ hohen Brechungsindex zwischen 1,65 und 1,75, insbesondere aus Polysulfon, gefertigt ist.

4. Verwendung eines intraokularen Implantates (1) mit einem optischen Abschnitt (2), der aus einer mittigen Zerstreuungslinse (4), deren Dicke von ihrem optischen Mittelpunkt (8) aus radial zunimmt, aus einer umfänglichen Einfassung (5), die einer Sammellinse entsprechend ausgebildet ist, und aus einem haptischen Abschnitt (3) zum Halten des optischen Abschnittes (2) gebildet wird, der beidseits desselben angeordnet ist, als Implantat zur Korrektur der Kurzsichtigkeit ohne Entfernung der Augenlinse nach Anspruch 1.
